# EUROPEAN PATENT APPLICATION

(11) **EP 4 528 286 A1**
(43) Date of publication of application: **26.03.2025**
(21) Application number: 23807254.0
(22) Date of filing: 07.03.2023
(51) Int. Cl.: G01N 35/08

(54) **SAMPLE ANALYZER**

(30) Priority: 17.05.2022 JP 2022080660
(71) Applicant: HITACHI HIGH-TECH CORPORATION, Tokyo 105-6409 (JP)
(72) Inventor: ITO, Hiroshi, Tokyo 100-8280 (JP); SUGIMURA, Yoshiaki, Tokyo 105-6409 (JP)
(74) Representative: Strehl & Partner mbB
(86) International application number: PCT/JP2023/008608
(87) International publication number: WO 2023/223638

(57) **Abstract**

An object of the present invention is to provide a sample analyzer that is less likely to cause positional deviation of a magnetic field in a channel regardless of an occurrence of positional deviation of a magnet and that enables highly accurate analysis. To attain this purpose, the present invention provides a sample analyzer including: a channel that introduces a sample liquid containing a magnetic particle bound to a labeling substance; a liquid feeding unit that supplies and discharges the sample liquid into and from the channel; a magnetic field applying unit that applies a magnetic field for capturing the magnetic particle in the channel; and a detector that detects the labeling substance bound to the magnetic particle that has been captured. The magnetic field applying unit includes a plurality of members, and at least one of the members is formed integrally with the channel.

## Description

### Technical Field

The present invention relates to a sample analyzer.

### Background Art

A sample analyzer such as an immunoassay analyzer introduces a sample liquid containing magnetic particles bound to a labeling substance into a channel such as a flow cell, captures (adsorbs) the magnetic particles in the channel, and applies an external electric field or the like to the captured magnetic particles to cause the labeling substance bound to the magnetic particles to emit light. The emission intensity at that time is detected, whereby an amount of the detection substance bound to the magnetic particles can be measured.

For example, PTL 1 describes a sample analyzer that collects magnetic particles by bringing a magnet close to a channel to strengthen a magnetic field when detecting emission intensity, and discharges the magnetic particles by moving the magnet away from the channel to weaken the magnetic field when cleaning the inside of the channel.

### Citation List

### Patent Literature

PTL 1: WO 2011/155489 A

### Summary of Invention

### Technical Problem

However, in a case where a mechanism in which a magnet as a magnetic field generation source moves is used, the number of components for holding the mechanism or operating the mechanism increases, and thus, the degree of freedom related to positioning increases, and when the mechanism is repeatedly used, positional deviation may occur. Even if the mechanism in which the magnet moves is not used, there is a possibility of positional deviation of the magnet when the components are operated for maintenance or the like. When a magnetic field in the channel deviates due to the positional deviation of the magnet, the magnetic particles cannot be reliably captured, so that the accuracy of analysis is reduced.

An object of the present invention is to provide a sample analyzer that is less likely to cause positional deviation of a magnetic field in a channel regardless of an occurrence of positional deviation of a magnet and that enables highly accurate analysis.

### Solution to Problem

To address the above-mentioned problem, the present invention provides a sample analyzer including: a channel that introduces a sample liquid containing a magnetic particle bound to a labeling substance; a liquid feeding unit that supplies and discharges the sample liquid into and from the channel; a magnetic field applying unit that applies a magnetic field for capturing the magnetic particle in the channel; and a detector that detects the labeling substance bound to the magnetic particle that has been captured, wherein the magnetic field applying unit includes a plurality of members, and at least one of the members is formed integrally with the channel.

### Advantageous Effects of Invention

The present invention can provide a sample analyzer that is less likely to cause positional deviation of a magnetic field in a channel regardless of an occurrence of positional deviation of a magnet and that enables highly accurate analysis.

### Brief Description of Drawings

[FIG. 1] FIG. 1 is a diagram illustrating a schematic configuration of an immunoassay analyzer.
[FIG. 2] FIG. 2 is a diagram illustrating configurations of a magnetic field applying unit and a flow cell according to a first embodiment.
[FIG. 3] FIG. 3 is a diagram illustrating a magnetic field generation source and the flow cell when positional deviation occurs.
[FIG. 4] FIG. 4 is a diagram illustrating an analysis model according to a comparative example that does not use a fixed magnetic material.
[FIG. 5] FIG. 5 is a diagram illustrating an analysis model according to the first embodiment that uses a fixed magnetic material.
[FIG. 6] FIG. 6 is a diagram showing analysis results of magnetic flux densities for the analysis model according to the comparative example and the analysis model according to the first embodiment.
[FIG. 7] FIG. 7 is a diagram illustrating an analysis model according to a second embodiment in which a fixed magnetic material is in contact with a core.
[FIG. 8] FIG. 8 is a diagram showing analysis results of magnetic flux densities for the analysis model according to the comparative example and the analysis model according to the second embodiment.
[FIG. 9] FIG. 9 is a diagram illustrating an analysis model according to a third embodiment in which an end face of the fixed magnetic material on the channel side has a smaller area.
[FIG. 10] FIG. 10 is a diagram showing analysis results of magnetic flux densities for the analysis model according to the comparative example and the analysis model according to the third embodiment.
[FIG. 11] FIG. 11 is a diagram illustrating a plurality of modifications regarding the shape of the fixed magnetic material.
[FIG. 12] FIG. 12 is a cross-sectional view illustrating a distribution of a magnetic flux density in a representative shape example of the fixed magnetic material.
[FIG. 13] FIG. 13 is a diagram illustrating configurations of a magnetic field applying unit and a flow cell according to a fourth embodiment.
[FIG. 14] FIG. 14 is a diagram illustrating an analysis model according to the fourth embodiment in which a fixed magnetic material is provided on a channel top wall.
[FIG. 15] FIG. 15 is a diagram showing analysis results of magnetic flux densities for the analysis model according to the comparative example and the analysis model according to the fourth embodiment.
[FIG. 16] FIG. 16 is a diagram illustrating configurations of a magnetic field applying unit and a flow cell according to a fifth embodiment.
[FIG. 17] FIG. 17 is a diagram illustrating configurations of a magnetic field applying unit and a flow cell according to a sixth embodiment.
[FIG. 18] FIG. 18 is a diagram illustrating configurations of a magnetic field applying unit and a flow cell according to a seventh embodiment.
[FIG. 19] FIG. 19 is a diagram illustrating configurations of a magnetic field applying unit and a flow cell according to an eighth embodiment.
[FIG. 20] FIG. 20 is a diagram illustrating configurations of a magnetic field applying unit and a flow cell according to a ninth embodiment.

### Description of Embodiments

A preferred embodiment of the present invention will be described below with reference to the drawings.

In the present preferred embodiment, an immunoassay analyzer will be described as an example of the sample analyzer. It is to be noted that the present invention is not limited to be used for immune assay, and can be applied to any sample analyzer as long as the sample analyzer captures magnetic particles by switching the magnetic field intensity using the magnetic particles. For example, the present invention can be applied to an analyzer for DNA assay, biochemical assay, or the like. In addition, the preferred embodiment described below is not limited in terms of shapes, dimensions, numbers, and the like, and can be modified without departing from the gist thereof.

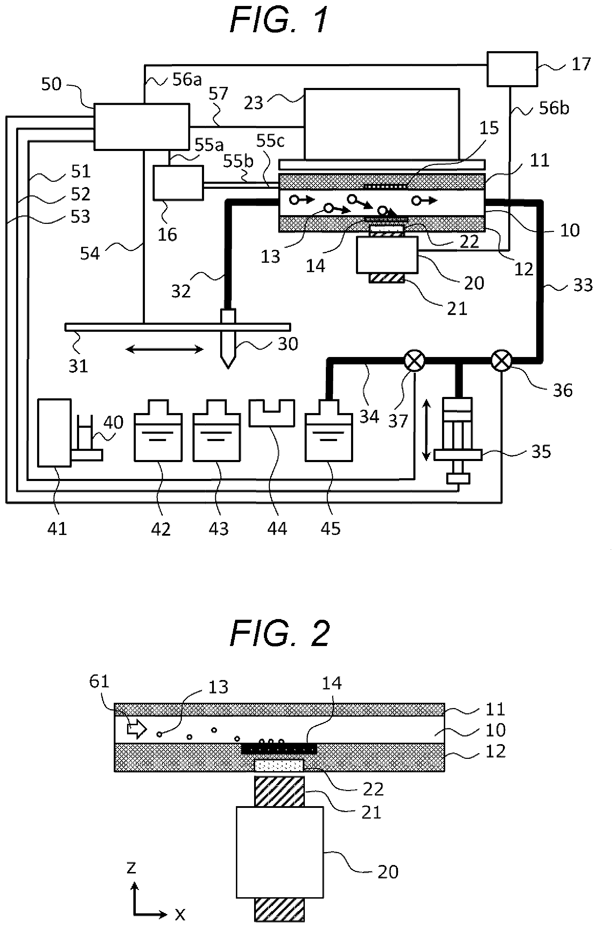

FIG. 1 is a diagram illustrating a schematic configuration of an immunoassay analyzer. The immunoassay analyzer includes a channel 10, a liquid feeding unit, a magnetic field applying unit, a photodetector 23, a controller 50, and the like. The channel 10 is a section into which a sample liquid containing magnetic particles 13 bound to a labeling substance is introduced, and is defined by a channel top wall 11 and a channel bottom wall 12. In the present specification, a detection unit including the channel top wall 11, the channel bottom wall 12, a reaction field electrode 14, and a counter electrode 15 is referred to as a flow cell as an integrated member.

The channel top wall 11 is preferably made of a transparent material such as glass or plastic. Thus, the photodetector 23 can detect the wavelength of light emitted from the labeling substance bound to the magnetic particles 13 around the reaction field electrode 14.

The reaction field electrode 14 is provided around the bottom of the channel 10, and the counter electrode 15 is provided to face the reaction field electrode 14 across the channel 10, that is, provided around the upper part of the channel 10. Further, the reaction field electrode 14 and the counter electrode 15 are connected to a voltage applying unit 16 via signal lines 55b and 55c. The voltage applying unit 16 is connected to the controller 50 via a signal line 55a.

The materials of the reaction field electrode 14 and the counter electrode 15 can be, for example, gold, platinum, palladium, tungsten, iridium, nickel, alloys thereof, carbon materials, and the like. Further, a material obtained by depositing the above materials into a film shape on a base material such as titanium by plating, sputtering, or the like, for example, can also be used as the reaction field electrode 14 and the counter electrode 15.

The reaction field electrode 14 basically has a planar shape in terms of ease of measurement of light emission, and is provided on the bottom surface of the channel 10. However, the reaction field electrode 14 may be provided on another surface in the channel 10 or on a plurality of surfaces arranged three-dimensionally. The reaction field electrode 14 is not limited to have a planar shape as long as it is suitable for capturing the magnetic particles 13 or for electrochemical luminescence of the labeling substance bound to the magnetic particles 13, and may have a linear shape, a combination of a linear shape and a planar shape, or the like. The counter electrode 15 may be provided at any position and may have any shape as long as it can generate a voltage in the channel 10 in combination with the reaction field electrode 14, and may have a linear shape, a planar shape, or a combination thereof.

In the present preferred embodiment, the magnetic field applying unit that applies a magnetic field for capturing the magnetic particles 13 in the channel 10 includes a magnetic field generation source and a fixed magnetic material 22 for inducing a magnetic field from the magnetic field generation source to the reaction field electrode 14. The magnetic field generation source may be an electromagnet or a permanent magnet.

In FIG. 1, an electromagnet including a coil 20 and a core 21 is used as the magnetic field generation source.

As the coil 20, a popular solenoid coil or the like can be used as long as a magnetic field can be generated as an electromagnet, and for example, a solenoid coil made of a solid or stranded copper wire can be used. In addition, popular soft magnetic materials such as iron, silicon steel, permalloy, permendur, magnetic alloy, or soft ferrite can be used as the core 21 and the fixed magnetic material 22 as long as a magnetic field can be generated or induced. The core 21 and the fixed magnetic material 22 may be formed of the same soft magnetic material or different soft magnetic materials.

The electromagnet including the coil 20 and the like is connected to the controller 50 via signal lines 56a and 56b and a voltage applying unit 17. When the magnetic particles 13 are captured (adsorbed), the voltage applying unit 17 is controlled by the controller 50, by which a voltage is applied to the coil 20. Thus, a magnetic field is generated around the reaction field electrode 14 and the counter electrode 15 in the channel 10. The magnetic particles 13 are collected on the reaction field electrode 14 by magnetic force derived from the generated magnetic field. When a voltage is applied to the coil 20, either a direct current or an alternate current may be used as long as the electromagnet including the coil 20 and the like can generate a magnetic field. When an alternate current is used, it is preferable to take a common measure against a skin effect such as using a stranded wire.

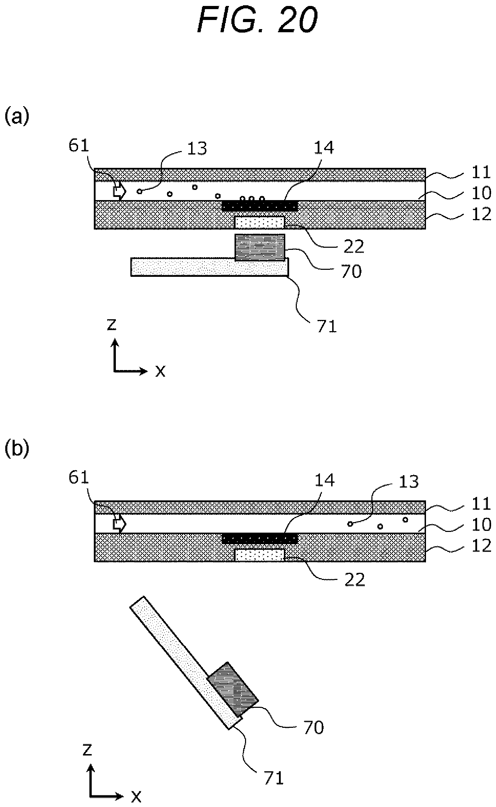

In a case where the magnetic field generation source is a permanent magnet, a movable arm and a permanent magnet, for example, are installed near the reaction field electrode 14, and the arm is moved by a signal from the controller 50 to change the distance between the permanent magnet and the reaction field electrode 14, as will be described later with reference to FIG. 20. According to this method, even when a permanent magnet is used as the magnetic field generation source, it is possible to control an increase and decrease of the magnetic field in the channel 10 as in the case of using the electromagnet.

The voltage applying unit 16 is controlled by the controller 50. When a voltage is applied between the reaction field electrode 14 and the counter electrode 15 in the channel 10 in response to a command from the controller 50 to the voltage applying unit 16, the labeling substance bound to the magnetic particles 13 captured on the reaction field electrode 14 can electrochemically emit light.

The photodetector 23 detects the labeling substance bound to the magnetic particles 13 captured by the magnetic field applied by the magnetic field applying unit, and is, for example, a camera, a photomultiplier tube, or the like. The photodetector 23 can detect a wavelength of light emitted from the labeling substance around the reaction field electrode 14, and operates by a signal from the controller 50.

The liquid feeding unit is a unit for supplying and discharging a sample liquid into and from the channel 10 in the flow cell, and includes a tube, a pump, a valve, and the like. The channel 10 is connected to a sipper nozzle 30 and a pump 35 through a tube 32 and a tube 33. The sipper nozzle 30 is attached to be movable by an arm 31, and a suspension container 40, a cleaning liquid container 42, and a buffer solution container 43 are installed within the range of movement of the sipper nozzle 30. The tube 33 between the channel 10 and the pump 35 is provided with a valve 36. The pump 35 and the valve 36 are connected to the controller 50 through signal lines 52 and 53, and are connected to a waste container 45 through a valve 37 and a tube 34.

The controller 50 is connected to the valves 36 and 37, the pump 35, the arm 31, the voltage applying units 16 and 17, and the photodetector 23 by independent signal lines 51, 52, 53, 54, 55a, 56a, and 57, respectively. As a result, the controller 50 can independently control the components connected thereto.

A sample to be analyzed is a substance derived from a living body such as serum or urine. When the sample is serum, a specific component to be analyzed is, for example, a tumor marker, an antibody, an antigen-antibody complex, or a single protein. In the following description, the specific component is a thyroid stimulating hormone (TSH).

The suspension container 40 stores a sample to be analyzed that is mixed with a bead solution and a reagent and then reacted at a certain temperature (for example, 37 degrees) for a certain period of time as a pretreatment process. The bead solution is prepared by dispersing the magnetic particles 13 in which a particulate magnetic substance is embedded in a matrix material such as polystyrene in a buffer solution, and streptavidin capable of binding to biotin is bound to the surface of the matrix material. The reagent contains a substance that binds the magnetic particles 13 to the specific component TSH in the sample, and includes an anti-thyroid stimulating hormone (TSH) antibody whose terminal is treated with biotin. The reagents vary depending on the type of specific component to be analyzed, and for example, immunoglobulins, antigens, antibodies, or other biological substances are used.

The cleaning liquid container 42 stores a cleaning liquid for cleaning the inside of the channel 10 and the tube 32.

When a length in the direction along the flow (path length) and a thickness (vertical direction) and a width (horizontal direction) of the cross section perpendicular to the flow are defined, the channel 10 preferably has a shape in which the path length is 2 to 20 times longer than the larger one of the thickness and the width. This is because, by sufficiently ensuring the path, the magnetic particles 13 in a fluid spread in the channel 10 and then are easily captured on the reaction field electrode 14 provided on the bottom surface of the channel 10.

The capture distribution of the magnetic particles 13 in the channel 10 is determined by a magnetic force received from the magnet (electromagnet or permanent magnet) installed in the vicinity of the channel 10 and the drag force due to the flow of the fluid. The magnetic field in the channel 10 preferably has a magnetic flux density of about 0.1 to 0.5 T. The flow velocity of the fluid at that time is preferably about 0.05 to 0.10 m/s.

The particles described below are preferably used as the magnetic particles 13.
(1) Particles exhibiting paramagnetism, superparamagnetism, ferromagnetism, or ferrimagnetism, and (2) particles obtained by encapsulating particles exhibiting paramagnetism, superparamagnetism, ferromagnetism, or ferrimagnetism in a material such as a synthetic polymer compound (polystyrene, nylon, etc.), a natural polymer (cellulose, agarose, etc.), or an inorganic compound (silica or the like). The particle size is preferably in the range of 0.01 to 200 µm, and more preferably in the range of 1 to 10 µm. The specific gravity is preferably 1.3 to 1.5. According to this specification, the magnetic particles 13 are less likely to settle in the liquid and are likely to be suspended. A substance having a property of specifically binding to the substance to be analyzed, for example, an antibody having a property of specifically binding to an antigen, is bound to the surface of the particle.

The substances described below are preferably used as the labeling substance. Specifically, the following substances are described as examples from the viewpoint of specifically binding the labeling substance to the substance to be analyzed by an appropriate means and allowing the labeling substance to emit light by an appropriate means.
(1) A labeling substance used in fluorescence immunoassay. For example, an antibody labeled with fluorescein isothiocyanate or the like.
(2) A labeling substance used in chemiluminescent immunoassay. For example, an antibody labeled with acridinium ester or the like.
(3) A labeling substance used in chemiluminescent enzyme immunoassay. For example, an antibody labeled with a chemiluminescent enzyme using luminol or an adamantyl derivative as a luminescent substrate.

The configuration of the immunoassay analyzer has been described above. Next, the operation of the immunoassay analyzer will be described. Regarding the operation, one analysis is defined as one cycle, and a state in which the immunoassay analyzer continuously performs the cycle a plurality of times is defined as a basic device operating state.

One cycle of analysis includes a suspension aspiration period, a magnetic particle capturing period, a detection period, a cleaning period, a reset period, and a preliminary aspiration period. One cycle is started when the suspension container 40 containing a suspension treated in a reaction unit 41 is set in a predetermined position.

During the suspension aspiration period, the valve 36 is opened and the valve 37 is closed. In response to a signal from the controller 50, the arm 31 is operated to insert the sipper nozzle 30 into the suspension container 40. Subsequently, the pump 35 performs a certain amount of aspiration operation in response to a signal from the controller 50. Aspirated by the fluid in the tube 32, the suspension in the suspension container 40 enters the tube 32 through the sipper nozzle 30. In this state, the pump 35 is stopped, and the arm 31 is operated to insert the sipper nozzle 30 into a cleaning mechanism 44. When passing through the cleaning mechanism 44, the sipper nozzle 30 is cleaned.

During the magnetic particle capturing period, a voltage is applied in response to a signal from the controller 50, so that the electromagnet including the coil 20 and the like operates. The pump 35 aspirates at a constant speed in response to a signal from the controller 50. Meanwhile, the suspension present in the tube 32 passes through the channel 10. A magnetic field by the electromagnet is generated in the channel 10, and thus, the magnetic particles 13 contained in the suspension are attracted toward the channel bottom wall 12 by magnetic force and captured on the reaction field electrode 14.

During the detection period, the application of voltage to the electromagnet is stopped in response to a signal from the controller 50. Subsequently, in response to a signal from the controller 50, the photodetector 23 is operated, and a voltage is applied to the reaction field electrode 14 and the counter electrode 15. Thus, light emitted from the labeling substance bound to the magnetic particles 13 is received by the photodetector 23 with the magnetic particles 13 being held on the reaction field electrode 14, whereby the measurement can be performed. The detected intensity of the emitted light is collected by the controller 50 as a signal. After a lapse of a certain time, the application of voltage to the reaction field electrode 14 and the counter electrode 15 and the operation of the photodetector 23 are stopped. During the detection period, the arm 31 is operated to insert the sipper nozzle 30 into the cleaning mechanism 44.

During the cleaning period, the cleaning liquid aspirated from the cleaning liquid container 42 passes through the channel 10 by aspiration using the pump 35. At this time, the electromagnet is in a non-operating state, whereby the magnetic particles 13 are not held on the reaction field electrode 14 and are washed away together with the cleaning liquid.

During the reset period, the valve 36 is closed, the valve 37 is opened, and the pump 35 performs a discharge operation. The liquid in the pump 35 is discharged to the waste container 45.

During the preliminary aspiration period, the buffer solution is aspirated from the buffer solution container 43, and the tube 32 and the channel 10 are filled with the buffer solution. After the preliminary aspiration period, the next one cycle can be started.

Regarding the sample analyzer according to the present preferred embodiment described above, a specific structure of the magnetic field applying unit will be described below by way of first to ninth embodiments.

### First Embodiment

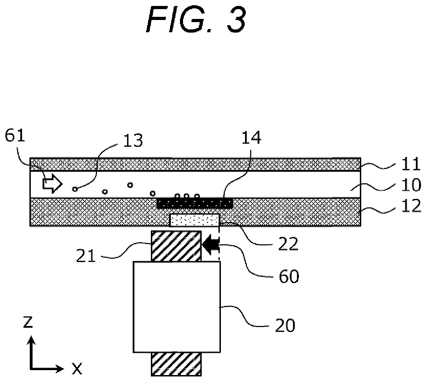

The structure of a sample analyzer according to the first embodiment will be described in detail with reference to FIGS. 2 and 3. In the present embodiment, an electromagnet is used as a magnetic field generation source. In sample analyzers, a magnetic field around the reaction field electrode 14 may vary due to individual differences in manufacturing, a change in use environment, a decrease in operating current due to heat generation, and the like. However, in the sample analyzer using an electromagnet as in the present embodiment, a desired magnetic field can be obtained without movement of the electromagnet by, for example, adjusting a load voltage (current) to the electromagnet or changing an operating time.

FIG. 2 is a diagram illustrating configurations of a magnetic field applying unit and a flow cell according to the first embodiment, and illustrates a cross section on a zx plane when a coordinate system is used in which a traveling direction of flow in the channel 10 is an x axis, a horizontal direction of the cross section perpendicular to the flow is a y axis, and the vertical direction thereof is a z axis. The magnetic particles 13 flow in the channel 10 of the flow cell along a flow direction 61. Note that FIG. 2 does not illustrate the counter electrode 15.

A magnetic field applying unit is provided below the reaction field electrode 14 of the flow cell. When a mechanism for moving the permanent magnet close to or away from the flow cell is used as the magnetic field applying unit, the number of components for holding the mechanism or operating the mechanism increases, and thus, the degree of freedom related to positioning increases, and when the mechanism is repeatedly used, positional deviation may occur. On the other hand, in a case where an electromagnet is used as the magnetic field applying unit as in the present embodiment, it is not necessary to move the magnetic field generation source, so that positional deviation is relatively less likely to occur. However, even in the case of using an electromagnet, the electromagnet may deviate when components are operated due to maintenance or the like.

In view of this, in the present embodiment, the magnetic field applying unit is constituted by a plurality of members, and a fixed magnetic material 22 among the components is integrally formed with the flow cell. Therefore, even when the magnetic field generation source (the coil 20 and the core 21), which is another member constituting the magnetic field applying unit, deviate, the fixed magnetic material 22 does not deviate, and thus, imbalance in the magnetic field around the reaction field electrode 14 is reduced.

In addition, the fixed magnetic material 22 is provided on the channel bottom wall 12, and the distance between the channel 10 and the fixed magnetic material 22 is minimum on the bottom surface side of the channel 10. That is, the fixed magnetic material 22 is interposed between the magnetic field generation source and the reaction field electrode 14, whereby the magnetic field generated by the magnetic field generation source can be effectively guided to the reaction field electrode 14.

Examples of a method for integrating the fixed magnetic material 22 with the flow cell include a method for fixing the fixed magnetic material 22 by bonding the fixed magnetic material to the channel wall with an adhesive or the like after the manufacture of the flow cell, and a method for fixing the fixed magnetic material 22 by embedding the fixed magnetic material in the channel wall by insert molding in the process of manufacturing the flow cell. When the fixed magnetic material 22 is joined to the flow cell so as to be exposed toward the magnetic field generation source, the fixed magnetic material 22 can be brought close to the magnetic field generation source, so that the magnetic flux leaking from the magnetic field generation source into the air can be reduced. Thus, a decrease in the magnetic field on the reaction field electrode 14 can be suppressed. On the other hand, in a case where the fixed magnetic material 22 is embedded in the channel bottom wall 12, the fixed magnetic material 22 is distant from the magnetic field generation source, so that the magnetic field reaching the fixed magnetic material 22 is weakened. However, the fixed magnetic material 22 is close to the channel 10, so that the deviation of the magnetic field with respect to the channel space is less likely to occur.

FIG. 3 is a diagram illustrating the magnetic field generation source and the flow cell when positional deviation occurs. The position of the electromagnet including the coil 20 and the core 21 disposed below the flow cell moves to the upstream side (-x direction) of the channel, so that positional deviation occurs. The distance from the end of the reaction field electrode 14 on the upstream (-x) side of the channel to the electromagnet decreases, while the distance from the end of the reaction field electrode 14 on the downstream (+x) side of the channel to the electromagnet increases. As a result, in a case where the fixed magnetic material 22 is not used, imbalance is caused in the magnetic field generated from the electromagnet by an operation of the electromagnet between the upstream side of the channel and the downstream side of the channel with respect to the reaction field electrode 14.

When the magnetic particles 13 are captured on the reaction field electrode 14, it is preferable to capture the magnetic particles 13 uniformly and at a high rate on the reaction field electrode 14. However, if imbalance is caused in the magnetic field around the reaction field electrode 14, the distribution or efficiency at the time of capturing the magnetic particles 13 deteriorates.

In view of this, in the present embodiment, the magnetic field generated from the electromagnet is guided to the reaction field electrode 14 via the fixed magnetic material 22 to thereby reduce imbalance in the magnetic field around the reaction field electrode 14. This makes it possible to suppress deterioration of distribution or efficiency at the time of capturing the magnetic particles 13 on the reaction field electrode 14 even when the magnet of the magnetic field generation source deviates.

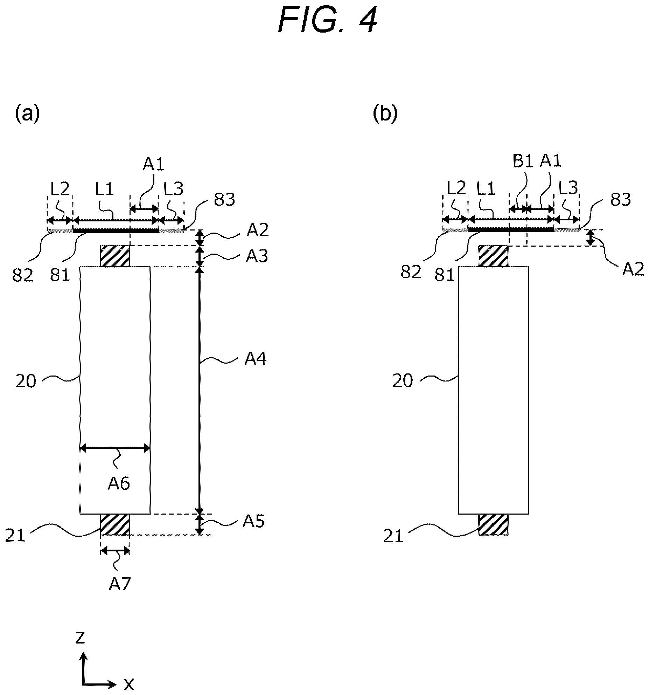

Regarding the effect of suppressing the positional deviation of the magnetic field according to the present embodiment, the results confirmed by magnetic field analysis will be described with reference to FIGS. 4, 5, and 6.

FIG. 4 illustrates an analysis model in which a fixed magnetic material is not used as a comparative example. FIG. 4(a) illustrates a case where the electromagnet including the coil 20 and the core 21 does not deviate, and FIG. 4(b) illustrates a case where the electromagnet deviates by a dimension B1 in the x direction from the state illustrated FIG. 4(a). A position 81 where the magnetic field is desired to be strengthened is indicated at a spatial position that is supposed to be a position on the reaction field electrode 14, and a channel-upstream-side adjacent portion 82 and a channel-downstream-side adjacent portion 83 which are adjacent to the position 81 are indicated. In FIG. 4(a), the center position of the position 81 where the magnetic field is desired to be strengthened on the xy plane is the same as the center position of the core 21 on the xy plane. Regarding the shape and dimension of the analysis model, the position and dimension of the core in the horizontal direction is represented by A1, the position and dimension of the core in the vertical direction is represented by A2, the upper core length is represented by A3, the coil length is represented by A4, the lower core length is represented by A5, the coil diameter is represented by A6, and the core diameter is represented by A7. Further, regarding the dimensions of the position 81 where the magnetic field is desired to be strengthened, the channel-upstream-side adjacent portion 82, and the channel-downstream-side adjacent portion 83, the x-axis dimension of the position where the magnetic field is desired to be strengthened is represented by L1, the x-axis dimension of the channel-upstream-side adjacent portion is represented by L2, and the x-axis dimension of the channel-downstream-side adjacent portion is represented by L3.

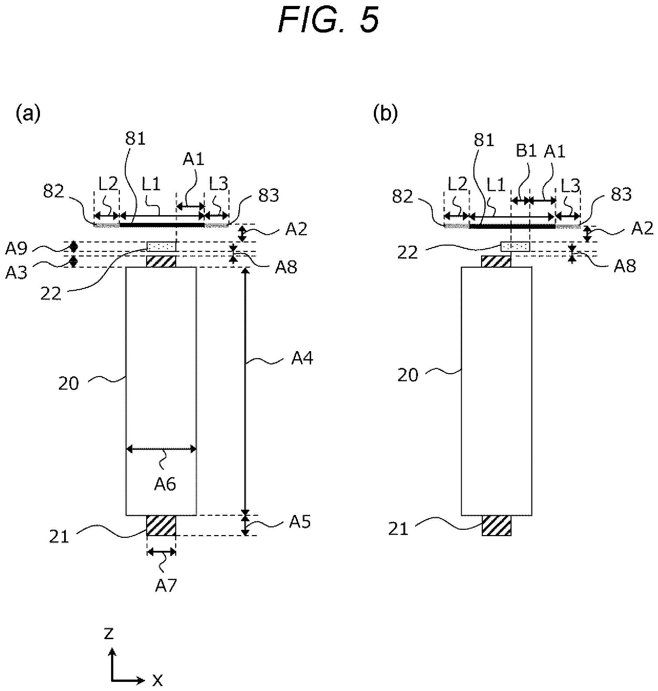

FIG. 5 illustrates an analysis model according to the first embodiment in which a fixed magnetic material is used. FIG. 5(a) illustrates a case where the electromagnet including the coil 20 and the core 21 does not deviate, and FIG. 5(b) illustrates a case where the electromagnet deviates by a dimension B1 in the x direction from the state illustrated FIG. 5(a). A position 81 where the magnetic field is desired to be strengthened is indicated at a spatial position that is supposed to be a position on the reaction field electrode 14, and a channel-upstream-side adjacent portion 82 and a channel-downstream-side adjacent portion 83 which are adjacent to the position 81 are indicated. Regarding the shape and dimension of the analysis model, the position and dimension of the core in the horizontal direction is represented by A1, the position and dimension of the core in the vertical direction is represented by A2, the upper core length is represented by A3, the coil length is represented by A4, the lower core length is represented by A5, the coil diameter is represented by A6, the core diameter is represented by A7, the gap between the fixed magnetic material and the core is represented by A8, and the length of the fixed magnetic material is represented by A9. Further, regarding the dimensions of the position 81 where the magnetic field is desired to be strengthened, the channel-upstream-side adjacent portion 82, and the channel-downstream-side adjacent portion 83, the x-axis dimension of the position where the magnetic field is desired to be strengthened is represented by L1, the x-axis dimension of the channel-upstream-side adjacent portion is represented by L2, and the x-axis dimension of the channel-downstream-side adjacent portion is represented by L3. The fixed magnetic material 22 is fixed to the flow cell, and thus, it can be seen from FIGS. 5(a) and 5(b) that only the coil 20 and the core 21 are moved with the relative position between the fixed magnetic material 22 and the position 81 where the magnetic field is desired to be strengthened being unchanged.

In FIGS. 4 and 5, the position and dimension A1 of the core in the horizontal direction is set to 0.5 mm, the position and dimension A2 of the core in the vertical direction is set to 0.5 mm, the upper core length A3 in FIG. 4 is set to 5 mm, the upper core length A3 in FIG. 5 is set to 3 mm, the coil length A4 is set to 60 mm, the lower core length A5 is set to 5 mm, the coil diameter A6 is set to 17 mm, the core diameter A7 is set to 7 mm, the positional deviation dimension B1 is set to 0 to 2 mm, the gap A8 between the fixed magnetic material and the core is set to 0.5 mm, and the length of the fixed magnetic material A9 is set to 2 mm. In addition, the x-axis dimension L1 of the position where the magnetic field is desired to be strengthened is set to 8 mm, the x-axis dimension L2 of the channel-upstream-side adjacent portion and the x-axis dimension L3 of the channel-downstream-side adjacent portion are set to 2 mm, and y-axis dimensions of the above position and portions are set to 8 mm in the direction into the page of FIGS. 4 and 5. Therefore, the position 81 where the magnetic field is desired to be strengthened is square with 8 mm, and the channel-upstream-side adjacent portion 82 and the channel-downstream-side adjacent portion 83 are rectangular with an x-axis dimension of 2 mm and a y-axis dimension of 8 mm.

The magnetic field analysis was performed by a versatile magnetic field analysis method based on Maxwell's equation and Ampere's law. The magnetic field analysis was performed as follows on the basis of the analysis models of the electromagnet illustrated in FIGS. 4 and 5. In a system having an air region of about five times longer than the long axis (sum of upper core length A3, coil length A4, and lower core length A5) of the electromagnet, an average magnetic flux density in each of the following three locations included in the air region was obtained, the three locations being the position 81 (square with an x-axis dimension and a y-axis dimension of 8 mm, respectively) where the magnetic field is desired to be strengthened, and the channel-upstream-side adjacent portion 82 and the channel-downstream-side adjacent portion 83 (rectangular with an x-axis dimension of 2 mm and a y-axis dimension of 8 mm). As an analysis condition, the coil 20 is formed from copper and has 5000 turns. For comparison, the current value is set to 0.5 A in FIG. 4 and 1.0 A in FIG. 5 such that the magnetic flux densities in FIGS. 4(a) and 5(a) are almost the same. PB permalloy, which is a kind of general permalloy, is used for the core 21 and the fixed magnetic material 22.

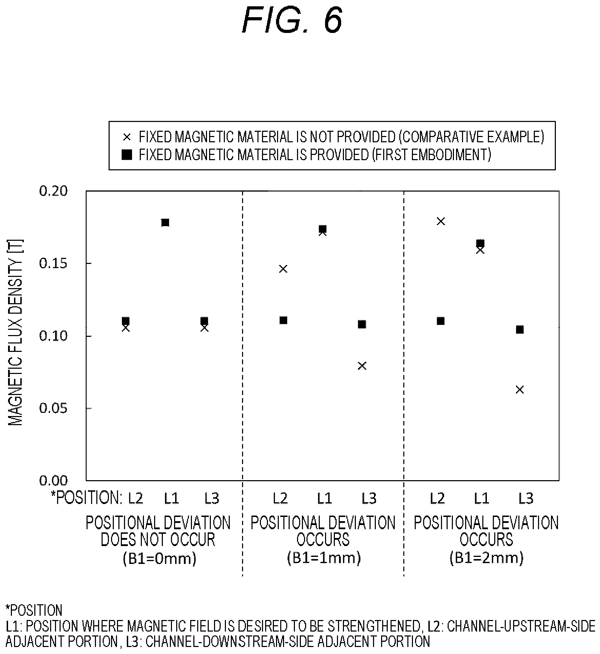

FIG. 6 is a diagram illustrating an analysis result of magnetic flux densities, and shows the magnitudes of the magnetic flux densities at three target locations for the analysis model according to the comparative example described with reference to FIG. 4 and the analysis model according to the first embodiment described with reference to FIG. 5. When positional deviation does not occur (B1 = 0 mm), a large magnetic flux density is generated at the position 81 where the magnetic field is desired to be strengthened, and a small magnetic flux density is generated at the channel-upstream-side adjacent portion 82 and the channel-downstream-side adjacent portion 83, regardless of the presence or absence of the fixed magnetic material. In this case, a strong magnetic field can be generated only on the reaction field electrode 14, and this magnetic field is considered to be a magnetic field suitable for capturing the magnetic particles 13. When positional deviation occurs (B1 = 1 mm or B1 = 2 mm), the magnetic flux density at the position 81 where the magnetic field is desired to be strengthened is high regardless of the presence or absence of the fixed magnetic material. In the comparative example, that is, in the model having no fixed magnetic material, the magnetic flux density increases particularly at the channel-upstream-side adjacent portion 82. When a strong magnetic field is generated even in a place other than the region on the reaction field electrode 14, the magnetic particles 13 are likely to be captured in a place that does not contribute to detection, and thus, such a magnetic field is considered to be an unsuitable magnetic field. On the other hand, in the present embodiment, that is, in the model having the fixed magnetic material, even if the position deviation occurs, the magnetic field smaller than that in the position 81 where the magnetic field is desired to be strengthened is maintained at the channel-upstream-side adjacent portion 82 and the channel-downstream-side adjacent portion 83. Therefore, by using the fixed magnetic material 22, a strong magnetic field limited in a region on the reaction field electrode 14 can be maintained, and a magnetic field structure in which the magnetic field around the reaction field electrode 14 is less likely to deviate can be provided.

Although the above analysis result shows only the effect in the x direction, the same effect can be expected also in the y direction and the z direction in the present embodiment that reduces imbalance in the magnetic field around the reaction field electrode 14 by guiding the magnetic field generated from the magnet to the reaction field electrode 14 with the fixed magnetic material 22. That is, even if the positional deviation of the magnet is caused by displacement in any direction in the xyz-space, inclination, or rotation, the magnetic field can be induced to an appropriate position in the space around the flow cell to reduce imbalance in the magnetic field by adjusting the position, size, shape, number, and the like of the fixed magnetic material 22. As a result, it is possible to provide a sample analyzer that is less likely to cause positional deviation of the magnetic field at a predetermined position in a detection channel and that enables highly accurate analysis.

### Second Embodiment

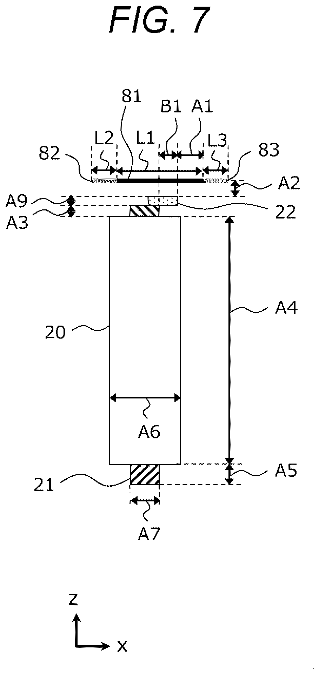

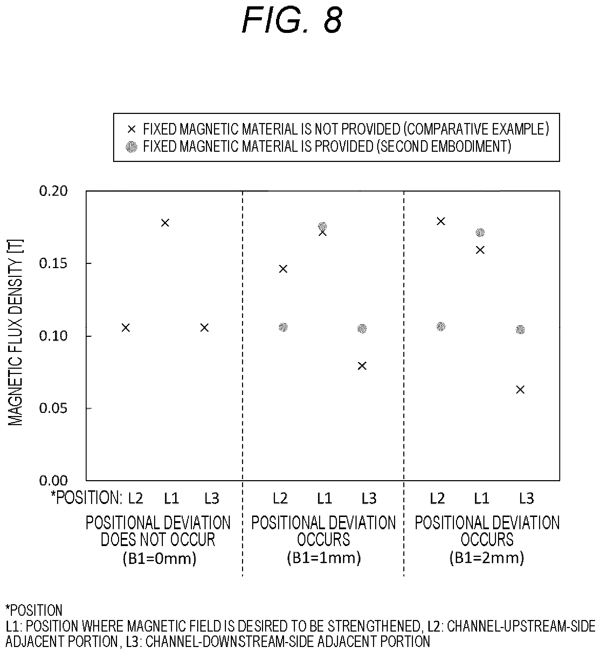

The second embodiment will be described with reference to FIGS. 7 and 8. In the following embodiments, the description of configurations and effects similar to those of the first embodiment will not be repeated.

FIG. 7 illustrates an analysis model according to the second embodiment in which a fixed magnetic material and a core are in contact with each other.

As illustrated in FIG. 7, a fixed magnetic material 22 according to the present embodiment is not only exposed from the bottom surface of a flow cell but also in contact with a core 21 of a magnetic field generation source. Therefore, it is possible to further reduce the magnetic flux leaking from the magnetic field generation source into the air, and to further suppress a decrease in the magnetic field on a reaction field electrode 14.

FIG. 7 illustrates a state in which a coil 20 and the core 21, which are not fixed to the flow cell, of a magnetic field applying unit including the coil 20, the core 21, and the fixed magnetic material 22 deviate. A position 81 where the magnetic field is desired to be strengthened is indicated at a spatial position that is supposed to be a position on the reaction field electrode 14, and a channel-upstream-side adjacent portion 82 and a channel-downstream-side adjacent portion 83 which are adjacent to the position 81 are indicated. Regarding the shape and dimension of the analysis model, the position and dimension of the core in the horizontal direction is represented by A1, the position and dimension of the core in the vertical direction is represented by A2, the upper core length is represented by A3, the coil length is represented by A4, the lower core length is represented by A5, the coil diameter is represented by A6, the core diameter is represented by A7, and the length of the fixed magnetic material is represented by A9. Further, the positional deviation dimension is represented by B1, the x-axis dimension of the position where the magnetic field is desired to be strengthened is represented by L1, the x-axis dimension of the channel-upstream-side adjacent portion is represented by L2, and the x-axis dimension of the channel-downstream-side adjacent portion is represented by L3.

In FIG. 7, the dimensions are set as described below for comparison with FIG. 4 (analysis model without having a fixed magnetic material). That is, the position and dimension A1 of the core in the horizontal direction is set to 0.5 mm, the position and dimension A2 of the core in the vertical direction is set to 0.5 mm, the upper core length A3 is set to 3 mm, the coil length A4 is set to 60 mm, the lower core length A5 is set to 5 mm, the coil diameter A6 is set to 17 mm, the core diameter A7 is set to 7 mm, the positional deviation dimension B1 is set to 0 to 2 mm, the length of the fixed magnetic material A9 is set to 2 mm, the x-axis dimension L1 of the position where the magnetic field is desired to be strengthened is set to 8 mm, and the x-axis dimension L2 of the channel-upstream-side adjacent position and the x-axis dimension L3 of the channel-downstream-side adjacent position are set to 2 mm (the position 81 where the magnetic field is desired to be strengthened is square with 8 mm, and the channel-upstream-side adjacent portion 82 and the channel-downstream-side adjacent portion 83 are rectangular with an x-axis dimension of 2 mm and a y-axis dimension of 8 mm). In addition, in FIG. 7, the core 21 and the fixed magnetic material 22 are arranged without a gap, and thus, the gap A8 between the fixed magnetic material and the core is 0 mm, and the description thereof is omitted.

The method for analyzing the magnetic field is similar to that in the first embodiment. Among various analysis conditions, the current value is set to 0.5 A that is the same as the current value in FIG. 4 in the present embodiment in FIG. 7. The other analysis conditions are similar to those in the first embodiment.

FIG. 8 is a diagram illustrating an analysis result of magnetic flux densities, and shows the magnitudes of the magnetic flux densities at three target locations for the analysis model according to the comparative example described with reference to FIG. 4 and the analysis model according to the second embodiment described with reference to FIG. 7. When positional deviation does not occur (B1 = 0 mm), the core 21 and the fixed magnetic material 22 in FIG. 7 are adjacent to each other without deviation, so that the structure of the analysis model is the same as that in FIG. 4. Therefore, plots of the model having the fixed magnetic material are not illustrated.

According to FIG. 8, when positional deviation occurs (B1 = 1 mm or B1 = 2 mm), the magnetic flux density at the channel-upstream-side adjacent portion 82 increases in the comparative example, that is, in the model having no fixed magnetic material, whereas the magnetic flux density is kept small in the present embodiment, that is, in the model having the fixed magnetic material. Therefore, by using the fixed magnetic material 22 according to the present embodiment, a magnetic field structure in which the magnetic field around the reaction field electrode 14 is less likely to deviate can be provided.

Furthermore, in the present embodiment, the gap A8 between the fixed magnetic material and the core is eliminated (or reduced), whereby it is possible to reduce a magnetic flux leaking from the gap into the air. As a result, it is possible to suppress a decrease in the magnetic field on the reaction field electrode 14 in addition to the effect that the positional deviation of the magnetic field around the reaction field electrode 14 is less likely to occur. Accordingly, in the present embodiment, it is not necessary to increase the applied voltage (current) as in the first embodiment in FIG. 5, and the positional deviation of the magnetic field around the reaction field electrode 14 is less likely to occur. Consequently, it is possible to ease manufacture and operation conditions and save consumption energy. For example, it is possible to reduce the turns of the coil 20, operate the coil 20 at low voltage (low current), or employ a material having a low magnetic susceptibility to the core 21 and the fixed magnetic material 22.

### Third Embodiment

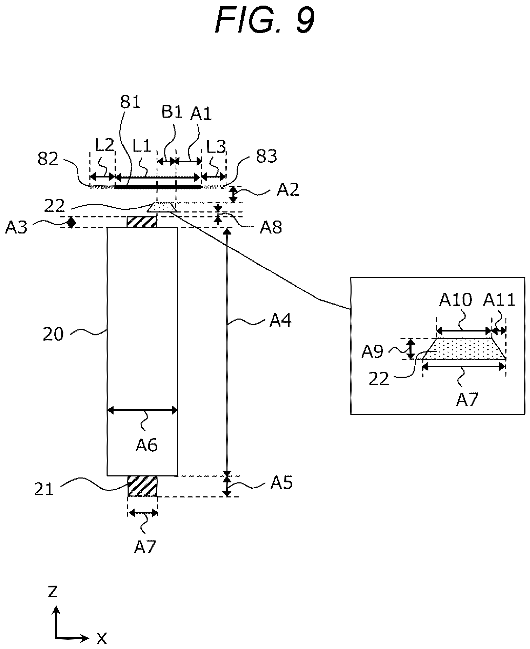

The third embodiment will be described with reference to FIGS. 9 to 12.

FIG. 9 illustrates an analysis model according to the third embodiment in which the end face of a fixed magnetic material on the channel side has a smaller area. As illustrated in FIG. 9, in the fixed magnetic material 22 according to the present embodiment, the cross-sectional area on the reaction field electrode side is smaller than the cross-sectional area on the magnetic field generation source side. For this reason, in the magnetic field generated by the magnetic field generation source, the density of the magnetic flux increases with nearness to the channel in the fixed magnetic material 22, and the force for capturing magnetic particles 13 increases.

FIG. 9 illustrates a state in which a coil 20 and a core 21, which are the magnetic field generation source, of a magnetic field applying unit including the coil 20, the core 21, and the fixed magnetic material 22 deviate. The fixed magnetic material 22 has a trapezoidal shape in an xz cross section in which the cross section at an end on the reaction field electrode 14 side (upper side) has a circular shape having a diameter smaller than that of the cross section at an end on the coil 20 side (lower side). A position 81 where the magnetic field is desired to be strengthened is indicated at a spatial position that is supposed to be a position on the reaction field electrode 14, and a channel-upstream-side adjacent portion 82 and a channel-downstream-side adjacent portion 83 which are adjacent to the position 81 are indicated. Regarding the shape and dimension of the analysis model, the position and dimension of the core in the horizontal direction is represented by A1, the position and dimension of the core in the vertical direction is represented by A2, the upper core length is represented by A3, the coil length is represented by A4, the lower core length is represented by A5, the coil diameter is represented by A6, the core diameter is represented by A7, the gap between the fixed magnetic material and the core is represented by A8, the length of the fixed magnetic material is represented by A9, the diameter of the upper surface of the fixed magnetic material is represented by A10, and the lateral dimension of the fixed magnetic material is represented by A11. Further, the positional deviation dimension is represented by B1, the x-axis dimension of the position where the magnetic field is desired to be strengthened is represented by L1, the x-axis dimension of the channel-upstream-side adjacent portion is represented by L2, and the x-axis dimension of the channel-downstream-side adjacent portion is represented by L3.

In FIG. 9, the dimensions are set as described below for comparison with FIG. 4 (analysis model without having a fixed magnetic material). That is, the position and dimension A1 of the core in the horizontal direction is set to 0.5 mm, the position and dimension A2 of the core in the vertical direction is set to 0.5 mm, the upper core length A3 is set to 3 mm, the coil length A4 is set to 60 mm, the lower core length A5 is set to 5 mm, the coil diameter A6 is set to 17 mm, the core diameter A7 is set to 7 mm, the positional deviation dimension B1 is set to 0 to 2 mm, the gap A8 between the fixed magnetic material and the core is set to 0.5 mm, the length of the fixed magnetic material A9 is set to 2 mm, the diameter A10 of the upper surface of the fixed magnetic material is set to 5 mm, the lateral dimension A11 of the fixed magnetic material is set to 1 mm, the x-axis dimension L1 of the position where the magnetic field is desired to be strengthened is set to 8 mm, and the x-axis dimension L2 of the channel-upstream-side adjacent position and the x-axis dimension L3 of the channel-downstream-side adjacent position are set to 2 mm (the position 81 where the magnetic field is desired to be strengthened is square with 8 mm, and the channel-upstream-side adjacent portion 82 and the channel-downstream-side adjacent portion 83 are rectangular with an x-axis dimension of 2 mm and a y-axis dimension of 8 mm).

The method for analyzing the magnetic field is similar to that in the first embodiment. Among various analysis conditions, the current value is set to 1.0 A that is the same as the current value in FIG. 5 in the present embodiment in FIG. 9. The other analysis conditions are similar to those in the first embodiment.

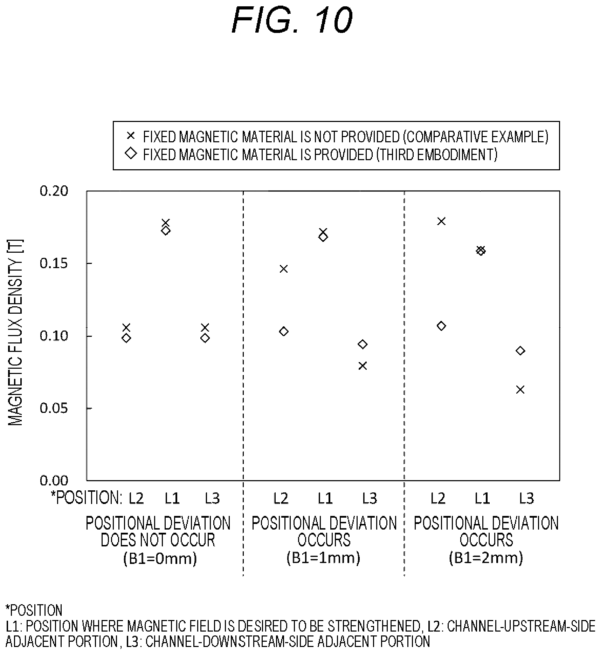

FIG. 10 is a diagram illustrating an analysis result of magnetic flux densities, and shows the magnitudes of the magnetic flux densities at three target locations for the analysis model according to the comparative example described with reference to FIG. 4 and the analysis model according to the third embodiment described with reference to FIG. 9. When positional deviation occurs (B1 = 1 mm or B1 = 2 mm), the magnetic flux density at the channel-upstream-side adjacent portion 82 increases in the comparative example, that is, in the model having no fixed magnetic material, whereas the magnetic field is kept small in the present embodiment, that is, in the model having the fixed magnetic material. Therefore, by using the fixed magnetic material 22 according to the present embodiment, a magnetic field structure in which the magnetic field around the reaction field electrode 14 is less likely to deviate despite an occurrence of positional deviation of the magnetic field generation source can be provided.

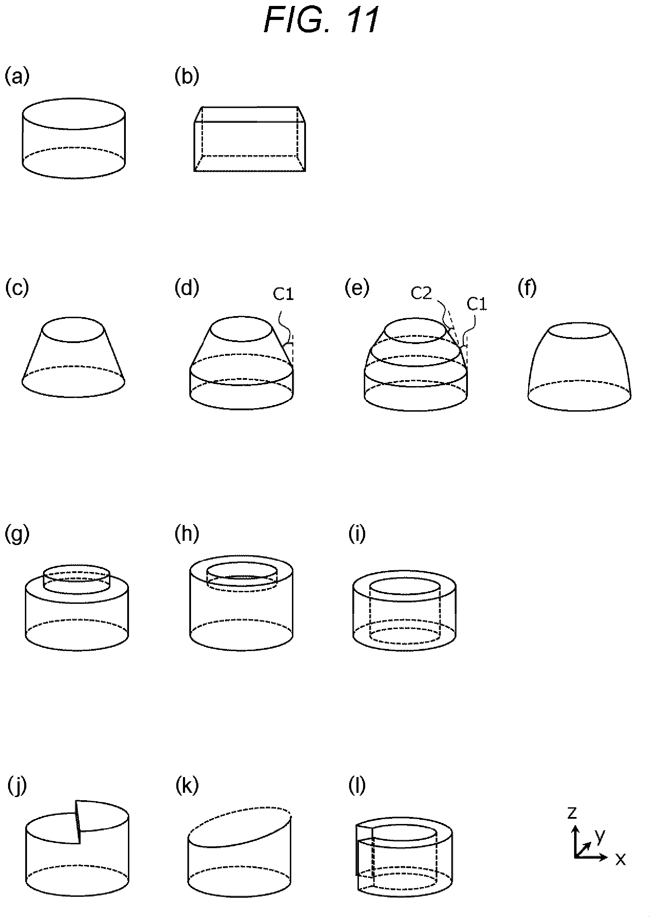

Note that various modifications are conceivable for the shape of the fixed magnetic material 22. Here, a plurality of representative shapes will be given as the shape of the fixed magnetic material 22, and individual effects obtained by each shape will be described. FIG. 11 is a diagram illustrating a plurality of modifications regarding the shape of the fixed magnetic material.

FIGS. 11(a) and (b) illustrate an example of the fixed magnetic material 22 having a cylindrical shape and an example of the fixed magnetic material 22 having a prismatic shape. The end face of the fixed magnetic material 22 on the reaction field electrode 14 side has a shape and size corresponding to the shape and size of the reaction field electrode 14, whereby the magnetic field on the reaction field electrode 14 can be efficiently increased. For example, in a case where the reaction field electrode 14 is square, a prismatic fixed magnetic material 22 having a square end face on the reaction field electrode 14 side is used, and in a case where the reaction field electrode 14 is circular, a cylindrical fixed magnetic material 22 having a circular end face on the reaction field electrode 14 side is used. Note that the end face of the fixed magnetic material 22 on the magnetic field generation source side may be formed to have a shape and size corresponding to the shape and size of the core 21 of the magnetic field generation source to reduce imbalance in the magnetic field.

FIGS. 11(c), (d), (e), and (f) illustrate examples in which the cross-sectional area at an end on the channel side (reaction field electrode 14 side) of the fixed magnetic material 22 is smaller than the maximum cross-sectional area of the fixed magnetic material 22. When the fixed magnetic material 22 induces a magnetic field, a magnetic flux passes from any one of the faces to the opposite face. For example, in the fixed magnetic material as illustrated in each of FIGS. 11(c), (d), (e), and (f), the magnetic flux entering the fixed magnetic material 22 from the face having a large cross-sectional area on the lower side in the z axis direction is emitted from the face having a small cross-sectional area on the upper side in the z axis direction, so that the density of the magnetic flux can be increased and a strong magnetic field can be generated in a limited range.

In addition, a large amount of magnetic flux entering the fixed magnetic material 22 from the core 21 via the air region is emitted from a sharp corner of the fixed magnetic material 22 to the air region. Therefore, the magnetic flux can be emitted into the air region from the corner on the side surface to reduce the magnetic flux not contributing to an increase in the magnetic field on the reaction field electrode 14 by using, for example, a shape in which an angle C1 on the side surface of the fixed magnetic material 22 is less than 90 degrees, more preferably 45 degrees or less, and still more preferably 30 degrees or less, as illustrated in FIG. 11(d). As a method for reducing sharp angles, two or more corners are formed on the side surface of the fixed magnetic material 22, and angles C1 and C2 on the side surface are set to be less than 90 degrees, more preferably 45 degrees or less, and still more preferably 30 degrees or less as illustrated in FIG. 11(e), for example. With this configuration, a similar effect can be expected. Furthermore, this effect is significant in a shape having no corner on the side surface, and can be further enhanced, for example, in a shape in which the side surface has a three-dimensional curved surface as illustrated in FIG. 11(f).

FIGS. 11(g), (h), (i), (j), and (l) illustrate examples in which the fixed magnetic material 22 has an uneven shape or hole on at least one end face of the fixed magnetic material 22.

By forming the upper end face of the fixed magnetic material 22 to have an uneven shape as illustrated in FIGS. 11(g), (h), and (i), it is possible to evenly provide a portion where a large amount of magnetic flux is emitted in the surface of the reaction field electrode 14 facing the fixed magnetic material by utilizing the fact that a large amount of magnetic flux entering the fixed magnetic material 22 is emitted from the corner at the upper end face to the air region. Thus, it is possible to reduce local imbalance in the magnetic field on the reaction field electrode 14 and to achieve uniform capturing of the magnetic particles 13 suitable for the detection of emitted light.

FIG. 11(j) illustrates an example in which the upper end face of the fixed magnetic material 22 has an uneven shape asymmetric on the upstream side and the downstream side of the channel 10 in the liquid feeding direction. In the sample analyzer, when the magnetic particles 13 are captured from a fluid, the capture distribution of the magnetic particles 13 on the reaction field electrode 14 may change depending on the flow velocity and the strength of the magnetic field. For example, under a condition where the flow is relatively slow, the magnetic particles 13 are likely to be captured on the upstream (-x) side of the reaction field electrode 14. In this case, in order to obtain the capture distribution of the magnetic particles 13 suitable for photodetection, the distance to the reaction field electrode 14 is increased on the upstream side of the fixed magnetic material 22, and the distance to the reaction field electrode 14 is decreased on the downstream (+x) side as illustrated in FIG. 11(j). With this configuration, the magnetic field is controlled so as to be weak on the upstream side of the reaction field electrode 14 and strong on the downstream side.

Thus, the capture distribution can be optimized so as to eliminate non-uniformity in the capture distribution of the magnetic particles 13 due to a flow field.

As the shape of the fixed magnetic material 22 having the same effect, for example, a shape in which the length of the side surface of the fixed magnetic material 22 varies depending on the location as illustrated in FIG. 11(k), and a shape in which the side surface of the fixed magnetic material 22 in a specific direction is cut off as illustrated in FIG. 11(l) are also effective. In FIG. 11(k), the magnetic flux passes asymmetrically on the upstream side and the downstream side of the channel 10 on the surface of the fixed magnetic material on which the magnetic flux is incident or the surface of the fixed magnetic material 22 from which the magnetic flux is emitted. With this configuration, the capture distribution can be optimized so as to eliminate non-uniformity in the capture distribution of the magnetic particles 13 due to the flow field. In addition, in the structure illustrated in FIG. 11(l), the side surface of the fixed magnetic material 22 is cut in a specific direction, and further, an uneven shape is formed in the end face. With this structure, it is possible to reduce local imbalance in the magnetic field on the reaction field electrode 14 in addition to the asymmetric generation of the magnetic field on the upstream side and the downstream side of the channel 10.

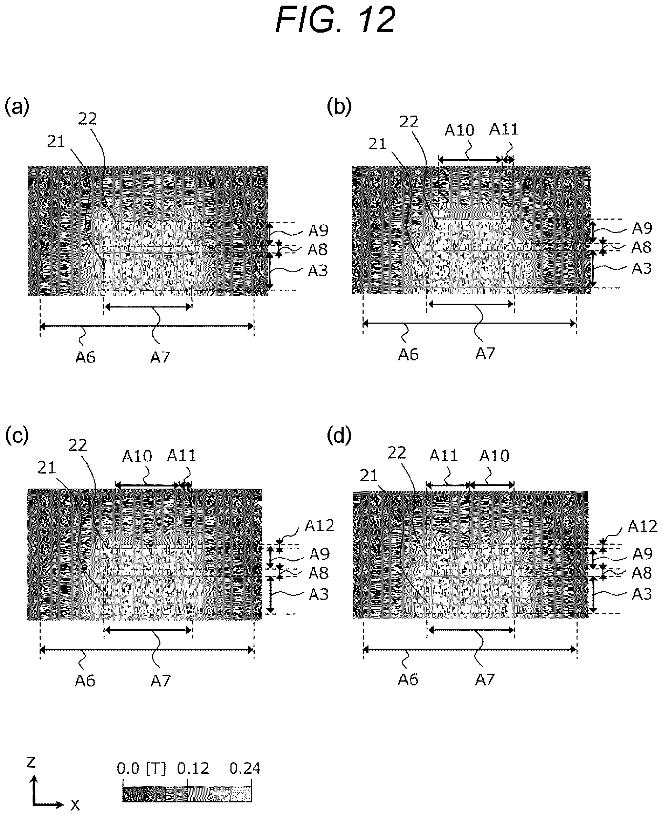

FIG. 12 is a cross-sectional view illustrating a distribution of a magnetic flux density in each of representative shape examples of the fixed magnetic material. FIGS. 12(a), (b), (c), and (d) respectively illustrate cases where the fixed magnetic material has: a cylindrical shape; a shape in which the cross-sectional area of the upper end part is smaller; a shape in which the upper end face has an uneven shape; and a shape in which the upper end face has an uneven shape that is asymmetrical, and these shapes correspond to the shapes illustrated in FIGS. 11(a), (c), (g), and (j), respectively.

FIGS. 12(a) and (b) illustrate the analysis cases same as that for the case of the positional deviation of 0 mm (B1 = 0 mm) in FIG. 5(a) and FIG. 9, and the dimensions are set as described below. That is, the position and dimension A1 of the core in the horizontal direction is set to 0.5 mm, the position and dimension A2 of the core in the vertical direction is set to 0.5 mm, the upper core length A3 is set to 3 mm, the coil length A4 is set to 60 mm, the lower core length A5 is set to 5 mm, the coil diameter A6 is set to 17 mm, the core diameter A7 is set to 7 mm, the positional deviation dimension B1 is set to 0 to 2 mm, the gap A8 between the fixed magnetic material and the core is set to 0.5 mm, the length of the fixed magnetic material A9 is set to 2 mm, the diameter A10 of the upper surface of the fixed magnetic material is set to 5 mm, and the lateral dimension A11 of the fixed magnetic material is set to 1 mm.

In FIG. 12(c), the length of the fixed magnetic material A9 is set to 1.7 mm, the diameter A10 of the upper surface of the fixed magnetic material is set to 5 mm, the lateral dimension A11 of the fixed magnetic material is set to 1 mm, the dimension A12 of a groove of the fixed magnetic material is set to 0.3 mm, and the other dimensions are the same as those in FIGS. 12(a) and (b).

In FIG. 12(d), the length of the fixed magnetic material A9 is set to 1.7 mm, the diameter A10 of the upper surface of the fixed magnetic material is set to 3.5 mm, the lateral dimension A11 of the fixed magnetic material is set to 3.5 mm, the dimension A12 of the groove of the fixed magnetic material is set to 0.3 mm, and the other dimensions are the same as those in FIGS. 12(a) and (b).

The method for analyzing the magnetic field is similar to that in the first embodiment. Among various analysis conditions, the current value is set to 1.0 A that is the same as the current value in FIG. 5 in the present embodiment in FIG. 12. The other analysis conditions are similar to those in the first embodiment.

The effects of the shape illustrated in FIG. 12(b) in which the cross-sectional area of the upper end face is smaller, the shape illustrated in FIG. 12(c) in which the upper end face has an uneven shape, and the shape illustrated in FIG. 12(d) in which the upper end face has an uneven shape that is asymmetrical will be described in comparison with the cylindrical shape illustrated in FIG. 12(a). Regarding the shape illustrated in FIG. 12(b) in which the cross-sectional area of the upper end face is smaller, the density of the magnetic flux generated from the central part of the upper end face of the fixed magnetic material 22 is larger than that in the fixed magnetic material having the cylindrical shape illustrated in FIG. 12(a), and the magnetic field is locally concentrated. As a result, the magnetic flux density limited on the reaction field electrode 14 can be increased. The effect of the shape illustrated in FIG. 12(c) in which the upper end face has an uneven shape is considered. The magnetic flux density generated from the upper part of the fixed magnetic material 22 is concentrated on the corners in the cylindrical shape illustrated in FIG. 12(a), whereas in the shape illustrated in FIG. 12 (c) in which the upper end face has an uneven shape, the concentration of the magnetic flux density on the corners is reduced, and the magnetic flux density tends to spread all over the entire upper surface of the fixed magnetic material 22. Thus, it is possible to reduce local imbalance in the magnetic field on the surface of the reaction field electrode 14 and to achieve uniform capturing of the magnetic particles 13 suitable for the detection of emitted light. Regarding the shape illustrated in FIG. 12(d) in which the upper end face has an uneven shape that is asymmetrical, the magnetic flux density generated from the upper part of the fixed magnetic material 22 varies in size between on the -x side and on the +x side. Thus, under a condition where, for example, the flow is relatively slow, the magnetic field on the downstream side of the reaction field electrode 14 is controlled to be greater than that on the upstream side, whereby it is possible to perform control so as to eliminate the non-uniformity in the capture distribution of the magnetic particles 13.

In addition, as can be seen from FIG. 12, in all shapes, the magnetic flux density is large from the corner of the upper end face of the fixed magnetic material 22 to a certain region. Therefore, even if the area of the reaction field electrode 14 located above the fixed magnetic material 22 is larger than the cross-sectional area of the upper end face of the fixed magnetic material 22 by a certain degree, it is possible to induce a large amount of magnetic flux over the entire region on the reaction field electrode 14 by using the magnetic flux reaching the reaction field electrode 14 from the fixed magnetic material 22 via the air region.

### Fourth Embodiment

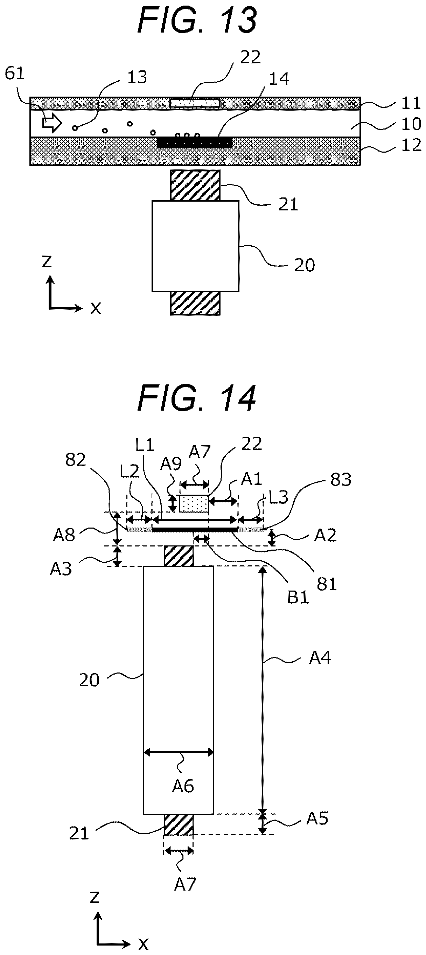

The fourth embodiment will be described with reference to FIGS. 13, 14, and 15.

FIG. 13 is a diagram illustrating configurations of a magnetic field applying unit and a flow cell according to the fourth embodiment, and illustrates a cross section on a zx plane when a coordinate system is used in which a traveling direction of flow in the channel 10 is an x axis, a horizontal direction of the cross section perpendicular to the flow is a y axis, and the vertical direction thereof is a z axis.

In the present embodiment, a fixed magnetic material 22 is provided on a channel top wall 11, and the distance between a channel 10 and the fixed magnetic material 22 is minimum on the top surface side of the channel 10. The present embodiment can also provide an effect that positional deviation of a magnetic field around a reaction field electrode 14 is less likely to occur because of the fixed magnetic material 22 being integrally formed with a flow cell. Although not illustrated in FIG. 13, a counter electrode 15 can be installed above the channel 10 without interfering with the fixed magnetic material 22 by, for example, arranging a plurality of linear electrodes in the x direction or the y direction in the channel 10.

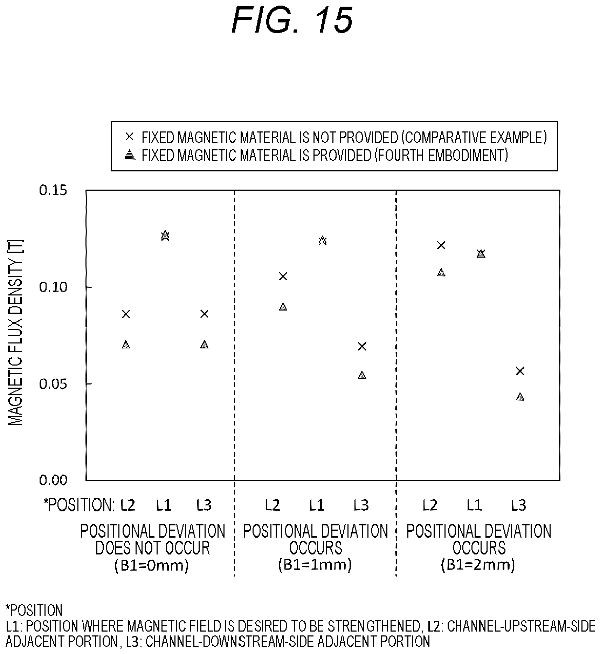

Regarding the effect of suppressing the positional deviation of the magnetic field according to the present embodiment, the results confirmed by magnetic field analysis will be described with reference to FIGS. 14 and 15.

FIG. 14 illustrates an analysis model according to the fourth embodiment in which the fixed magnetic material is provided on the channel top wall. In FIG. 14, a position 81 where the magnetic field is desired to be strengthened is indicated, assuming that the reaction field electrode 14 is present in a space between the core 21 and the fixed magnetic material 22. Further, at adjacent positions, a channel-upstream-side adjacent portion 82 and a channel-downstream-side adjacent portion 83 are indicated. FIG. 14 illustrates a state in which the coil 20 and the core 21 deviate with a positional deviation dimension of B1. Regarding the shape and dimension of the analysis model, the position and dimension of the core in the horizontal direction is represented by A1, the position and dimension of the core in the vertical direction is represented by A2, the upper core length is represented by A3, the coil length is represented by A4, the lower core length is represented by A5, the coil diameter is represented by A6, the core diameter is represented by A7, the gap between the fixed magnetic material and the core is represented by A8, and the length of the fixed magnetic material is represented by A9. Further, the positional deviation dimension is represented by B1, the x-axis dimension of the position where the magnetic field is desired to be strengthened is represented by L1, the x-axis dimension of the channel-upstream-side adjacent portion is represented by L2, and the x-axis dimension of the channel-downstream-side adjacent portion is represented by L3.

In FIG. 14, the gap A8 between the fixed magnetic material and the core is 3 mm, and the position 81 where the magnetic field is desired to be strengthened is set at the center thereof, and thus, the position and dimension A2 of the core in the vertical direction is set to 1.5 mm. In FIG. 15, an analysis result of a model in which the position 81 where the magnetic field is desired to be strengthened is set at a location corresponding to the location in FIG. 4 (analysis model without having a fixed magnetic material) with the position and dimension A2 of the core in the vertical direction being set as 1.5 mm is used for comparison. In FIG. 14, the length of the fixed magnetic material A9 is set to 5 mm, and the other dimensions are the same as those in FIG. 4.

The method for analyzing the magnetic field is similar to that in the first embodiment, and the current value is set to 0.5 A in FIG. 4 and set to 0.35 A in FIG. 14 so that the magnetic flux densities in FIGS. 4(a) and 14 are almost the same. The other analysis conditions are similar to those in the first embodiment.

FIG. 15 is a diagram illustrating an analysis result of magnetic flux densities, and shows the magnitudes of the magnetic flux densities at three target locations for the analysis model according to the comparative example described with reference to FIG. 4 and the analysis model according to the fourth embodiment described with reference to FIG. 14. When positional deviation does not occur (B1 = 0 mm), the magnetic flux density at the position 81 where the magnetic field is desired to be strengthened is substantially equal between the model without having a fixed magnetic material, that is, the comparative example, and the model having the fixed magnetic material provided on the channel top wall, that is, the present embodiment. However, the magnetic flux density at the channel-upstream-side adjacent portion 82 and at the channel-downstream-side adjacent portion 83 in the present embodiment is smaller than that in the comparative example. Therefore, in the present embodiment, a strong magnetic field can be limited only on the reaction field electrode 14.

When positional deviation occurs (B1 = 1 mm or B1 = 2 mm), a difference between the magnetic flux density at the position 81 where the magnetic field is desired to be strengthened and the magnetic flux density at the channel-upstream-side adjacent portion 82 and a difference between the magnetic flux density at the position 81 where the magnetic field is desired to be strengthened and the magnetic flux density at the channel-downstream-side adjacent portion 83 are greater in the present embodiment having the fixed magnetic material provided on the channel top wall than in the comparative example having no fixed magnetic material. Therefore, the present embodiment is more effective in generating a magnetic field limited on the reaction field electrode 14, and can ensure a magnetic field suitable for capturing the magnetic particles 13. The present embodiment can also provide a magnetic field structure in which the magnetic field around the reaction field electrode 14 is less likely to deviate regardless of an occurrence of positional deviation of the magnetic field generation source.

In a case where the fixed magnetic material 22 is provided on the channel top wall, the magnetic flux easily passes through the space between the core 21 and the fixed magnetic material 22, so that a larger magnetic flux density is easily obtained than in a case where the fixed magnetic material is not provided.

This is advantageous for capturing the magnetic particles 13, and capturing of the magnetic particles suitable for photodetection can be achieved.

Alternatively, a load voltage (current) to the coil 20 necessary for obtaining the equivalent magnetic flux density can be reduced, and the operation with energy saving can be achieved.

In a case where the fixed magnetic material is disposed on the upper side as in the present embodiment, the fixed magnetic material 22 may interfere with an optical path when light emission in the flow cell is observed by the photodetector 23. In that case, a hole is formed in the fixed magnetic material 22, for example, as in the shape illustrated in FIG. 11(i) or (l), by which the optical path can be ensured.

### Fifth Embodiment

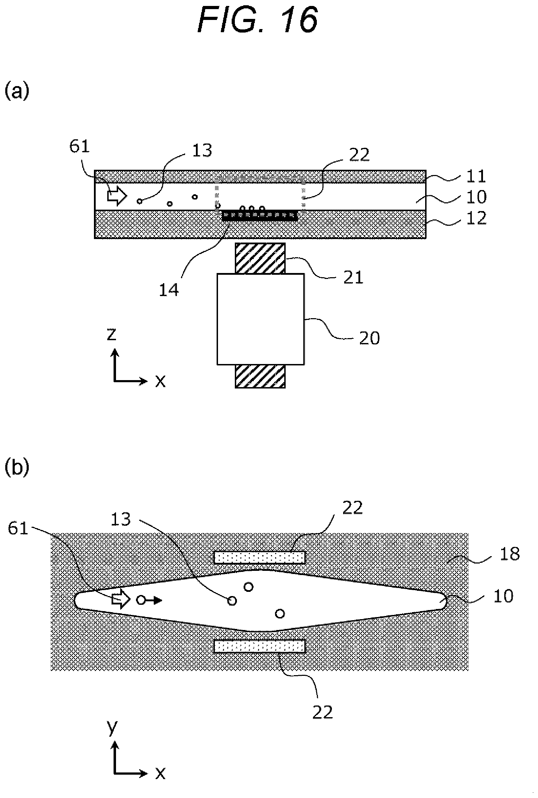

The fifth embodiment will be described with reference to FIG. 16. FIG. 16 is a diagram illustrating a configuration of a magnetic field applying unit and a flow cell according to the fifth embodiment. When a coordinate system is used in which a traveling direction of flow in the channel 10 is an x axis, a horizontal direction of the cross section perpendicular to the flow is a y axis, and the vertical direction thereof is a z axis, FIG. 16(a) illustrates a cross section on a zx plane, and FIG. 16(b) illustrates a cross section on an xy plane.

In the present embodiment, a fixed magnetic material 22 is disposed on the side part of a channel 10, that is, on a channel side wall 18. Such an arrangement can also provide a magnetic field structure in which the positional deviation of the magnetic field around the reaction field electrode 14 is less likely to occur. When the fixed magnetic material 22 is provided on the channel side wall 18, it may be provided on one or both of the channel side walls 18 facing each other.

### Sixth Embodiment

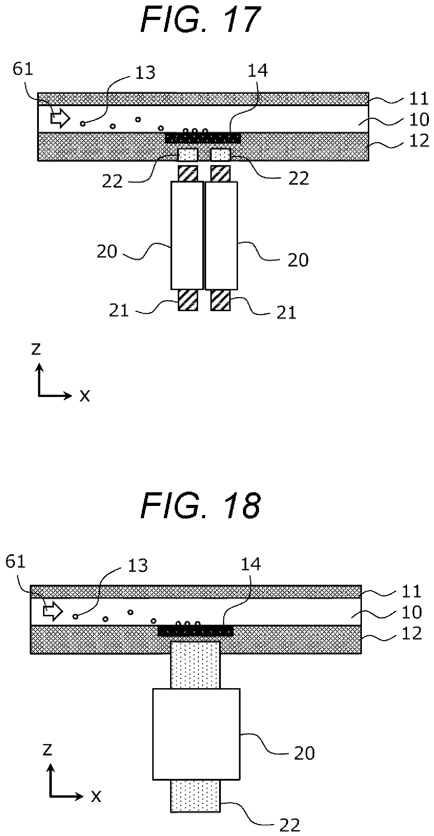

The sixth embodiment will be described with reference to FIG. 17. FIG. 17 is a diagram illustrating a configuration of a magnetic field applying unit and a flow cell according to the sixth embodiment. In the present embodiment, a plurality of the fixed magnetic materials 22 is provided. Such an arrangement can also provide a magnetic field structure in which the positional deviation of the magnetic field around the reaction field electrode 14 is less likely to occur.

Furthermore, the magnetic field on the reaction field electrode 14 can be appropriately controlled by using a plurality of fixed magnetic materials 22 which is different in size, shape, material, or the like as in the present embodiment. For example, the fixed magnetic material 22 having a small magnetic susceptibility or saturation magnetic flux density is disposed in the vicinity of the upstream side of the reaction field electrode 14, and the fixed magnetic material 22 having a large magnetic susceptibility or saturation magnetic flux density is disposed in the vicinity of the downstream side of the reaction field electrode 14, whereby the magnetic field can be asymmetrically controlled. With this configuration, nonuniformity in the capture distribution of the magnetic particles 13 due to the flow field can be eliminated. In addition, the magnetic field on the upstream side and on the downstream side of the reaction field electrode 14 can also be controlled using specifications or operating conditions of the coil 20 and the core 21 of the plurality of electromagnets. For example, a voltage (current) larger than that applied to the coil 20 of the electromagnet in the vicinity of the upstream side of the reaction field electrode 14 is applied to the coil 20 of the electromagnet in the vicinity of the downstream side of the reaction field electrode 14, by which the magnetic field in the vicinity of the downstream side can be increased.

Therefore, it is not necessary to divide both the fixed magnetic material 22 and the coil 20 and the core 21 that constitute the magnetic field generation source into a plurality of parts, and only the fixed magnetic material 22 may be divided into multiple parts or only the magnetic field generation source may be divided into multiple parts. Note that, in a case where only one fixed magnetic material 22 is used, the fixed magnetic material 22 may have a shape as illustrated in FIG. 11(j) or (k), for example.

### Seventh Embodiment

The seventh embodiment will be described with reference to FIG. 18. FIG. 18 is a diagram illustrating a configuration of a magnetic field applying unit and a flow cell according to the seventh embodiment. In the present embodiment, a fixed magnetic material is used without using a core, that is, a fixed magnetic material 22 integrated with a flow cell can be inserted into an internal space of a coil 20. Therefore, the fixed magnetic material 22 according to the present embodiment has a function of generating a magnetic field in addition to a function of inducing a magnetic field. The present embodiment can also provide a magnetic field structure in which the positional deviation of the magnetic field around a reaction field electrode 14 is less likely to occur.

### Eighth Embodiment

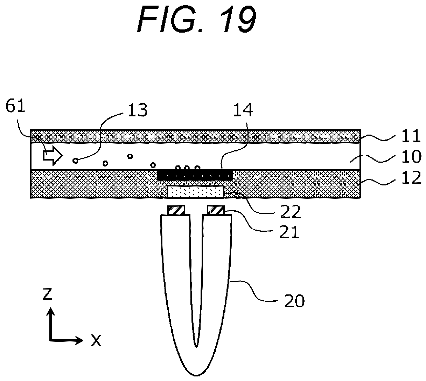

The eighth embodiment will be described with reference to FIG. 19. FIG. 19 is a diagram illustrating configurations of a magnetic field applying unit and a flow cell according to the eighth embodiment. In the present embodiment, an electromagnet having a bent shape is used, both ends of a core 21 face a flow cell, and a fixed magnetic material 22 is provided to face both ends of the core 21. This configuration can provide a magnetic field structure in which the positional deviation of the magnetic field around a reaction field electrode 14 is less likely to occur.

Furthermore, by using an electromagnet having a bent shape as in the present embodiment, it is possible to create a magnetic circuit with less air region around the flow cell, whereby the magnetic flux density generated from the end of the core 21 can be increased. Since the fixed magnetic material 22 faces both ends of the core 21, a large magnetic flux density can be obtained on the reaction field electrode 14 via a large amount of magnetic flux. In the present embodiment, even when the reaction field electrode 14 is on the channel bottom wall 12, the electromagnet is not limited to be placed below the flow cell. That is, an electromagnet having a bent shape may be disposed, for example, from below the flow cell to the side surface thereof, from one side surface to the opposing side surface, or from below to an area above the flow cell.

### Ninth Embodiment

The ninth embodiment will be described with reference to FIG. 20. FIG. 20 is a diagram illustrating configurations of a magnetic field applying unit and a flow cell according to the ninth embodiment. In the present embodiment, a permanent magnet 70 is used as a magnetic field generation source. Specifically, a movable arm 71 to which the permanent magnet 70 is fixed is rotated to bring the permanent magnet 70 close to or away from the flow cell to thereby increase or decrease the magnetic field on a reaction field electrode 14.

FIG. 20(a) illustrates a state in which the permanent magnet 70 is close to the flow cell, and this state is established when magnetic particles 13 are captured on the reaction field electrode 14. On the other hand, FIG. 20(b) illustrates a state in which the permanent magnet 70 is away from the flow cell, and this state is established when the magnetic particles 13 are removed from the reaction field electrode 14.

In the present embodiment, the fixed magnetic material 22 is formed integral with the flow cell, and thus, a magnetic field structure in which positional deviation of a magnetic field around the reaction field electrode 14 is less likely to occur can be obtained. Note that the movable arm 71 in the present embodiment is not limited to a rotary motion mechanism with one supporting point, and regarding the number, position, and direction of supporting points during motion, any number of supporting points may be set based on any position and any plane in a three-dimensional space. In addition, as a motion mechanism using motions other than rotation, another mechanism using, for example, a motion on one axis using a rail or the like may be adopted.

The present invention is not limited to the above-described embodiments, and various modifications are possible. For example, the above-described embodiments have been described in detail for easy understanding of the present invention, and are not necessarily limited to those having all the described configurations. In addition, a part of the configuration of a certain embodiment can be replaced with or added to the configuration of another embodiment.

### Reference Signs List

10 channel
11 channel top wall
12 channel bottom wall
13 magnetic particle
14 reaction field electrode
15 counter electrode
16, 17 voltage applying unit
18 channel side wall
20 coil
21 core
22 fixed magnetic material
23 photodetector
30 sipper nozzle
31 arm
32, 33, 34 tube
35 pump
36, 37 valve
40 suspension container
41 reaction unit
42 cleaning liquid container
43 buffer solution container
44 cleaning mechanism
45 waste container
50 controller
51, 52, 53, 54, 55a, 55b, 55c, 56a, 56b, 57 signal line
61 flow direction
70 permanent magnet
71 movable arm
81 position where magnetic field is desired to be strengthened
82 channel-upstream-side adjacent portion
83 channel-downstream-side adjacent portion

## Claims

1. A sample analyzer comprising:
a channel that introduces a sample liquid containing a magnetic particle bound to a labeling substance;
a liquid feeding unit that supplies and discharges the sample liquid into and from the channel;
a magnetic field applying unit that applies a magnetic field for capturing the magnetic particle in the channel; and
a detector that detects the labeling substance bound to the magnetic particle that has been captured, wherein
the magnetic field applying unit includes a plurality of members, and at least one of the members is integrally formed with the channel.

2. The sample analyzer according to claim 1, wherein
the channel is provided with an electrode to which a voltage for causing the labeling substance to emit light is applied, and
the magnetic field applying unit includes a magnetic field generation source and a magnetic material that induces a magnetic field from the magnetic field generation source to the electrode, the magnetic material being integrally formed with the channel.

3. The sample analyzer according to claim 2, wherein
the magnetic field generation source is an electromagnet including a coil.

4. The sample analyzer according to claim 2, wherein
the magnetic field generation source is a permanent magnet.

5. The sample analyzer according to claim 2, wherein
the magnetic material is embedded in a wall of the channel.

6. The sample analyzer according to claim 2, wherein
the magnetic material is exposed from the channel toward the magnetic field generation source.

7. The sample analyzer according to claim 6, wherein
the magnetic material is in contact with the magnetic field generation source.

8. The sample analyzer according to claim 2, wherein
the electrode includes a reaction field electrode provided at a bottom part of the channel and a counter electrode provided at an upper part of the channel, and
the magnetic field generation source is located below the reaction field electrode.

9. The sample analyzer according to claim 8, wherein
the magnetic material is provided on a bottom wall of the channel.

10. The sample analyzer according to claim 9, wherein
the magnetic material has a shape in which an end face on a side of the reaction field electrode is along a shape of the reaction field electrode, or a shape in which an end face on a side of the magnetic field generation source is along a shape of the magnetic field generation source.

11. The sample analyzer according to claim 9, wherein
the magnetic material has a smaller cross-sectional area on the side of the reaction field electrode than a cross-sectional area on the side of the magnetic field generation source.

12. The sample analyzer according to claim 9, wherein
the magnetic material has an end face smaller than the reaction field electrode on a side of the reaction field electrode.

13. The sample analyzer according to claim 8, wherein
the magnetic material is provided on a top wall of the channel.

14. The sample analyzer according to claim 2, wherein
the magnetic material has an asymmetric shape on an upstream side and on a downstream side of the channel in a liquid feeding direction.

15. The sample analyzer according to claim 2, wherein
different magnetic materials are provided on an upstream side and a downstream side of the channel in a liquid feeding direction.

## Amended claims

### Amended claims under Art. 19.1 PCT

1. (Amended) A sample analyzer comprising:
a channel that introduces a sample liquid containing a magnetic particle bound to a labeling substance;
a liquid feeding unit that supplies and discharges the sample liquid into and from the channel;
a magnetic field applying unit that applies a magnetic field for capturing the magnetic particle in the channel; and
a detector that detects the labeling substance bound to the magnetic particle that has been captured, wherein
the channel is provided with an electrode to which a voltage for causing the labeling substance to emit light is applied, and
the magnetic field applying unit includes a magnetic field generation source and a magnetic material that induces a magnetic field from the magnetic field generation source to the electrode, the magnetic material being integrally formed with the channel.

2. (Canceled)

3. (Amended) The sample analyzer according to claim 1, wherein
the magnetic field generation source is an electromagnet including a coil.

4. (Amended) The sample analyzer according to claim 1, wherein
the magnetic field generation source is a permanent magnet.

5. (Amended) The sample analyzer according to claim 1, wherein
the magnetic material is embedded in a wall of the channel.

6. (Amended) The sample analyzer according to claim 1, wherein
the magnetic material is exposed from the channel toward the magnetic field generation source.

7. The sample analyzer according to claim 6, wherein
the magnetic material is in contact with the magnetic field generation source.

8. (Amended) The sample analyzer according to claim 1, wherein
the electrode includes a reaction field electrode provided at a bottom part of the channel and a counter electrode provided at an upper part of the channel, and
the magnetic field generation source is located below the reaction field electrode.

9. The sample analyzer according to claim 8, wherein
the magnetic material is provided on a bottom wall of the channel.

10. The sample analyzer according to claim 9, wherein
the magnetic material has a shape in which an end face on a side of the reaction field electrode is along a shape of the reaction field electrode, or a shape in which an end face on a side of the magnetic field generation source is along a shape of the magnetic field generation source.

11. The sample analyzer according to claim 9, wherein
the magnetic material has a smaller cross-sectional area on the side of the reaction field electrode than a cross-sectional area on the side of the magnetic field generation source.

12. The sample analyzer according to claim 9, wherein
the magnetic material has an end face smaller than the reaction field electrode on a side of the reaction field electrode.

13. The sample analyzer according to claim 8, wherein
the magnetic material is provided on a top wall of the channel.

14. (Amended) The sample analyzer according to claim 1, wherein
the magnetic material has an asymmetric shape on an upstream side and on a downstream side of the channel in a liquid feeding direction.

15. (Amended) The sample analyzer according to claim 1, wherein
different magnetic materials are provided on an upstream side and a downstream side of the channel in a liquid feeding direction.
Statement under Art. 19.1 PCT
Claim 1 after amendment is obtained by adding the recitation of claim 2 before amendment to claim 1 before amendment.
Claim 2 is canceled.
Claims 3-6, 8, and 14-15 are amended to cite claim 1 instead of claim 2.
